# EUROPÄISCHE PATENTSCHRIFT

(11) **EP 0 312 844 B1**
(45) Veröffentlichungstag und Bekanntmachung des Hinweises auf die Patenterteilung: **22.06.1994**
(21) Anmeldenummer: 88116570.8
(22) Anmeldetag: 04.04.1985
(51) Int. Cl.: C07D 417/12

(54) **Cephalosporinzwischenprodukte, Verfahren zu ihrer Herstellung und ihre Verwendung**
Cephalosporin intermediates, process for their preparation and their use
Intermédiaires de céphalosporine, procédé pour leur préparation et leur application

(30) Priorität: 10.04.1984 AT 1197/84; 10.04.1984 AT 1198/84
(43) Veröffentlichungstag der Anmeldung: 26.04.1989
(62) Teilanmeldung aus: 85902014.1
(73) Patentinhaber: BIOCHEMIE GESELLSCHAFT M.B.H., 6250 Kundl (AT)
(72) Erfinder: Ascher, Gerd, A-6250 Kundl (AT); Sturm, Herbert, A-6500 Landeck (AT); Thaler, Heinrich, A-6322 Kirchbich 195 (AT); Veit, Werner, A-6330 Kufstein (AT)
(74) Vertreter: Kleine-Deters, Johannes

(56) Entgegenhaltungen:
- EP-A- 0 037 380

## Beschreibung

Unsere EP-B-0 185 679 betrifft ein Verfahren zur Herstellung von Cephalosporinzwischenprodukten der Formel 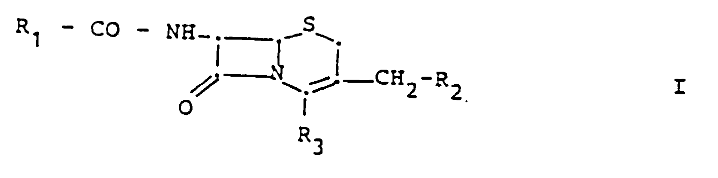
worin R₁ für die Gruppe der Formel 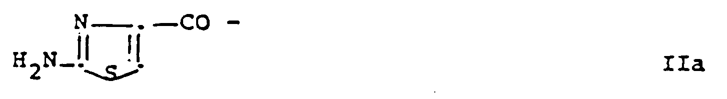
steht, R₂ für Wasserstoff, die Acetoxy-, die Carbamoyloxy-, eine S-Y-Gruppe, wobei Y einen unsubstituierten oder substituierten Heterocyclus bedeutet, oder R₂ für ein gegebenenfalls substituiertes Pyridinium der Formel 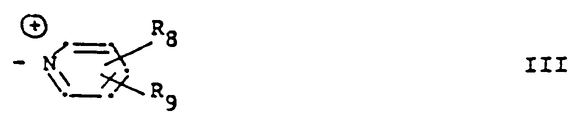
wobei R₈ und R₉ gleich oder verschieden sind und jeweils für Wasserstoff, Halogen, Alkyl, Hydroxy, Carboxamido, Alkoxycarbonyl, Amino, Monoalkylamino oder Dialkylamino stehen oder zusammen einen gegebenenfalls substituierten, 5- oder 6-gliedrigen carbocyclischen Ring bedeuten, steht und R₃ die Carboxyl-, die Carboxylat- oder eine Carbonsäureestergruppe bedeutet, und ihre Verwendung.

Weiters betrifft die EP-B-0 185 679 die neuen CephalosporinzwiSchenprodukte der Formel 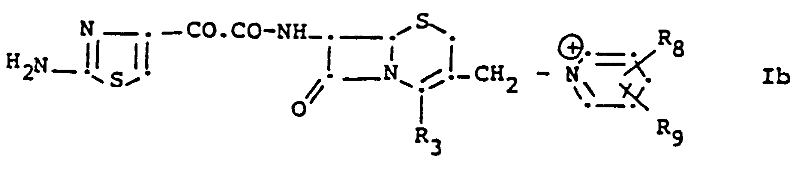
worin R₃, R₈ und R₉ obige Bedeutung besitzen.

Aus den Cephalosporinzwischenprodukten der Formel I können wertvolle Cephalosporinantibiotika hergestellt werden. Verbindungen, worin R₁ eine Gruppe der Formel II bedeutet, sind durch das Vorhandensein einer Oximinogruppe in der am Cephalosporinkern gebundenen 7-Acylamidoseitenkette charakterisiert. Diese Oximinogruppe kann in syn- oder anti-Konfiguration vorliegen, wobei die Verbindungen mit syn-Konfiguration bevorzugt sind.

Der heterocyclische Ring in R₇ beinhaltet, wie angeführt, ein oder mehrere Sauerstoff-, Schwefel- und/oder Stickstoffatome als Heteroatom(e). Er kann aber auch zusätzlich ein oder mehrere Stickstoffatome enthalten. Geeignete Heterocyclen sind beispielsweise Pyrazolyl, Furyl, Thienyl, Thiazolyl, Thiadiazolyl, Oxazolyl und Oxadiazolyl. Der Heterocyclus kann unsubstituiert oder durch Amino substituiert sein. Vorzugsweise ist der Heterocyclus von R₇ Thiazolyl, vorzugsweise durch Amino substituiert. In diesen Verbindungen kann R₂ Wasserstoff sein. Es kann auch Carbamoyloxy sein. Vorzugsweise bedeutet es aber Acetoxy, -S-Y oder gegebenen falls substituiertes Pyridinium. Geeignete Heterocyclen als Y sind bekannt. Bevorzugte Heterocyclen sind beispielsweise Thiadiazolyl, Diazolyl, Triazolyl, Tetrazolyl, Thiazolyl, Thiatriazolyl, Oxazolyl, Oxadiazolyl, Triazolylpyridyl, Purinyl, Pyridyl, Pyrimidinyl, Pyridazinyl, Pyrazolyl und Triazinyl. Diese Heterocyclen können unsubstituiert oder beispielsweise bis dreifach substituiert sein. Als Substituenten sind (C₁₋₄)Alkyl, (C₁₋₄)Alkoxy, Halogen, Trihalo-(C₁₋₄)alkyl, Hydroxy, Oxo, Mercapto, Amino, Carboxyl, Carbamoyl, Di-(C₁₋₄)alkylamino, Carboxymethyl, Carbamoylmethyl, Sulfomethyl und Methoxycarbonylamino geeignet. In der Literatur bisher als bevorzugt beschriebene Heterocyclen beinhalten Tetrazolyl, insbesondere 1-Methyl-1H-tetrazol-5-yl, und Triazinyl, insbesondere 1,2,5,6-Tetrahydro-2-methyl-5,6-dioxo-as-triazin-3-yl, 2,5-Dihydro-6-hydroxy-2-methyl-5-oxo-as-tiazin-3-yl oder 1,4,5,6-Tetrahydro-4-methyl-5,6-dioxo-as-triazin-3-yl. Vorzugsweise ist R₂ Acetoxy, 1-Methyl-1H-tetrazol-5-ylthio, 2,5-Dihydro-6-hydroxy-2-methyl-5-oxo-as-triazin-3-ylthio oder Pyridinium. Wie auf dem Gebiet der Cephalosporine bekannt, können die Verbindungen in Form der freien Säuren (R₃ = COOH) oder von Salzen, beispielsweise von Alkali- oder Erdalkalimetallsalzen, vorzugsweise von Alkalimetallsalzen, wie Natriumsalzen, vorliegen. Weiters können die Verbindungen auch in Form von Estern, beispielsweise in Form des Pivaloyloxymethylesters vorliegen, aber auch als andere Ester, wie beispielsweise Acetoxymethyl-, 1-Acetoxyethyl-, 1-Ethoxycarbonyloxyethyl-, 5-Indanoyl- oder vorzugsweise Hexanoylmethyl-, Phthalidyl-, Carbethoxymethoxymethyl- oder 3-Carbethoxy-1-acetonylester. Steht R₂ für eine gegebenenfalls substituierte Pyridiniumgruppe, so können die Verbindungen der Formel I auch als innere Salze vorliegen. Weitere R₂-Gruppen, die basische Reste enthalten, können ebenfalls in Form von Salzen vorliegen.

Insbesondere umfasst die EP-B-0 185 679 ein Verfahren zur Herstellung von Verbindungen der Formel I und ihrer Salze, dadurch gekennzeichnet, daß man eine Verbindung der Formel 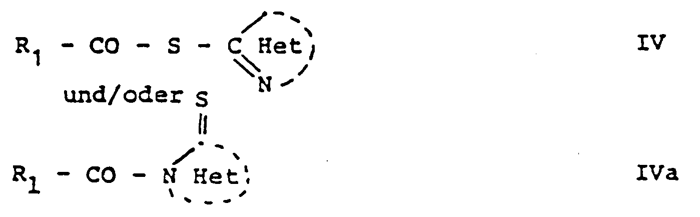
worin R₁ obige Bedeutung besitzt, und 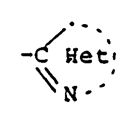
bzw. 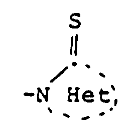
für einen 5- oder 6-gliedrigen Heterocyclus, der zusätzlich zum N-Atom ein oder zwei weitere Heteroatome ausgewählt aus 0, N und S beinhalten kann, und der mit einem Benzolring annelliert sein kann, stehen, mit einer Verbindung der Formel 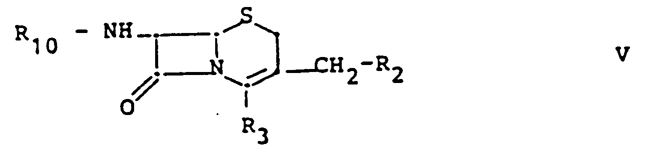
worin R₂ und R₃ obige Bedeutung besitzen und R₁₀ für Wasserstoff oder eine Aminoschutzgruppe steht, umsetzt und gewünschtenfalls ein erhaltenes Endprodukt entschützt und gegebenenfalls ein erhaltenes Endprodukt, worin R₃ für COOH steht, in ein Salz überführt oder umgekehrt.

Die Verbindungen der Formel IV können auch in Form der Formel IVa vorliegen, wobei das Verhältnis der beiden Formen zueinander von den Reaktionspartnern und den Reaktionsbedingungen abhängig ist. Wenn Verbindungen der Struktur der Formel IV angeführt werden, so umfassen diese immer auch Verbindungen der Struktur der Formel IVa.

Das Verfahren wird vorzugsweise in einem inerten Lösungsmittel oder in einem Gemisch eines solchen Lösungsmittels mit Wasser, z. B in einem chlorierten Kohlenwasserstoff, wie Dichlormethan, oder in einem Säureester, wie Essigsäureethylester, oder in Azeton, Dimethylformamid oder Dimethylsulfoxid bei einer Temperatur von -40 bis +60° C, insbesondere bei -15 bis +25° C und vorteilhafterweise bei 0 bis 20° C ausgeführt. Die Reaktionsdauer beträgt etwa 1/2 bis 48 Stunden. Die Reaktanden der Formel IV und V werden entweder in stöchiometrischen Mengen eingesetzt oder es wird ein Überschuß von bis zu 25 % an Verbindung der Formel IV verwendet.

Bei der Herstellung von Verbindungen der Formel I, worin R₃ COOH bedeutet, wie auch ihrer Salze kann die Carboxylgruppe im Ausgangsprodukt der Formel V geschützt sein. Geeignete Schutzgruppen sind bekannt und umfassen nicht nur die oben angeführten Bedeutungen, sondern auch Silylesterschutzgruppen, insbesondere die Trimethylsilylschutzgruppe, die z. B. durch Reaktion der freien Säure mit N,O-Bis(trimethylsilyl)acetamid eingeführt werden kann.

Die 7-Aminogruppe des Ausgangsmaterials der Formel V kann ebenfalls geschützt sein. Geeignete Schutzgruppen sind bekannt und umfassen z. B. die Trimethylsilylgruppe, die beispielsweise gleichzeitig mit Schützung der Carboxylgruppe eingeführt werden kann.

Der Aminosubstituent im Ausgangsmaterial IV/IVa kann in freier oder geschützter Form voliegen. Wie bereits ausgeführt, ist im allgemeinen ein Schutz nicht notwendig. Sollte jedoch trotzdem ein Schutz erwünscht sein, so kann dies in bekannter Weise, unter Verwendung geeigneter bekannter Schutzgruppen, durchgeführt werden.

Nach der Reaktion der Verbindungen der Formel IV und V können nachfolgende Entschützungsreaktionen in bekannter Weise durchgeführt werden. Ebenso kann die Überführung der freien Säureform (R₃ = COOH) in die Salze nach an sich bekannten Methoden ausgeführt werden.

Die Endprodukte können nach bekannten Methoden isoliert und gereinigt werden.

Im erfindungsgemäßen Verfahren verwendet man als reaktives Derivat der Säure der Formel A heterocyclische Thioester. Es wurde überraschenderweise gefunden, daß diese Ester hergestellt und verwendet werden können. Weiters wurde überraschenderweise gefunden, daß eine im Heterocyclus dieser Ester vorhandene Aminogruppe nicht zur Selbstreaktion führt. Demzufolge ist ein Schützen dieser Aminogruppe in der folgenden Acylie rung nicht notwendig. Andererseits kann die Aminogruppe selbstverständlich geschützt werden, falls dies aus einem anderen Grund gewünscht wird.

Die vorliegende Erfindung betrifft die neuen Verbindungen der Formel IV oder IVa. Bei den Verbindungen der Formel IV ist die Art des Ringes 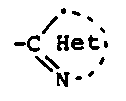
bzw. 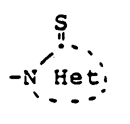
nicht kritisch, die bevorzugten Verbindungen werden durch Faktoren, wie Leichtigkeit bei der Herstellung und Verfügbarkeit des Ausgangsmaterials bestimmt. Vorzugsweise bedeutet der Ring 2-Pyridyl oder insbesondere 2-Benzothiazolyl. Er kann auch für Pyrimidinyl, Triazolyl oder Thiazolyl stehen.

Die Verbindungen der Formel IV/IVa können durch Veresterung von Verbindungen der Formel

R₁ - COOH VI

worin R₁ obige Bedeutung besitzt, erhalten werden.

Die Veresterung kann beispielsweise durch Reaktion mit einer Verbindung der Formel 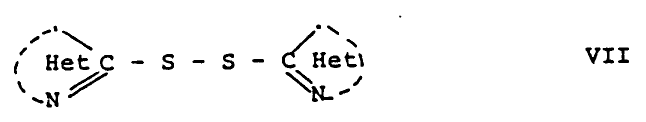
worin beide 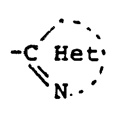
gleich sind und obige Bedeutung besitzen, durchgeführt werden.

Diese Veresterung wird vorzugweise unter Gegenwart eines Tri(niederalkyl)-oder Tri(aryl)phosphins oder -phosphits, insbesondere Triphenylphosphins, durchgeführt, und zwar vorzugsweise bei einer Temperatur von -30 bis +50° C, insbesondere bei -20 bis +25° C und vorteilhafterweise bei -5 bis +5° C. Als Lösungsmittel wird ein inertes, nicht hydroxylgruppenhältiges organisches Lösungsmittel, z. B. ein chlorierter Kohlenwasserstoff, wie Dichlormethan, eingesetzt. Wird eine Verbindung der Formel IV gewünscht, worin R₁ für einen Heterocyclus mit einer geschützten Aminogruppe steht, so kann die Aminoschutzgruppe vor oder nach der Veresterung eingeführt werden. Die Endprodukte können durch Behandeln mit niederen Alkoholen kristallisiert und gereinigt werden, wobei darauf geachtet werden muß, daß die Temperatur 20° C, vorzugsweise 0° C, nicht übersteigt.

In den nachfolgenden Beispielen, die die Erfindung näher erläutern, ihren Umfang aber in keiner Weise einschränken sollen, erfolgen alle Temperaturangaben in Celsiusgraden.

### Beispiel 1 : 2-(2-Aminothiazol-4-yl)-2-oxothioessigsäure-S-benzothiazol-2-ylester:

34,4 g Aminothiazolylglyoxylsäure werden in 500 ml Dichlormethan suspendiert und unter Rühren mit 28 ml Triethylamin versetzt. Man kühlt auf -15° und gibt 64 g Triphenylphosphin und 80 g Bisbenzothiazol-2-yldisulfid zu. Nach zweistündigem Rühren bei -15° wird der orange Niederschlag abfiltriert, mit kaltem Dichlormethan gewaschen und im Vakuum getrocknet. Ausbeute: 57,7 g, d.i. 89,8 %. IR: 3300 N-H, 1675 Carbonyl (Thioester), 1645 Carbonyl (Amid), 1540 Amidbande.

### Beispiel 2 : 2-(2-Aminothiazol-4-yl)-2-oxothioessigsäure-S-benzothiazol-2-ylester:

80 g Bisbenzothiazol-2-yldisulfid und 64 g Triphenylphosphin werden in 500 ml Tetrahydrofuran suspendiert und auf -15° gekühlt. Man gibt 34,4 g Aminothiazolylglyoxylsäure zu und tropft 16,1 ml Pyridin so langsam zu, daß die Innentemperatur nicht über -15° ansteigt. Die orange Suspension wird zwei Stunden bei -15° gerührt. Der Niederschlag wird abfiltriert, mit kaltem Tetrahydrofuran gewaschen und im Vakuum getrocknet. Ausbeute: 62,2 g als Tetrahydrofuran-Solvat. Fp: bei 80° Entweichen von THF, ab 123° (Zers.). THF-Gehalt (gaschromatographisch): 17 %

## Patentansprüche

1. Cephalosporinzwischenprodukte der Formel IV oder IVa 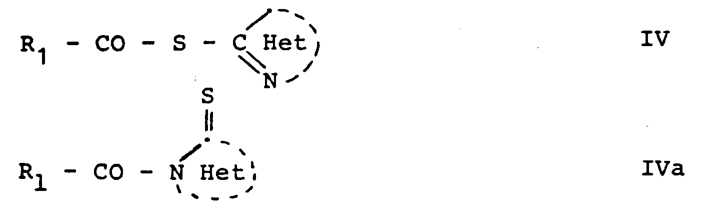 worin R₁ für die Gruppe der Formel 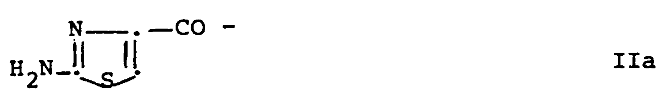 steht und 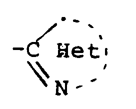 bzw. 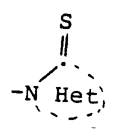 für einen 5- oder 6-gliedrigen Heterocyclus stehen, der zusätzlich zum N-Atom ein oder zwei weitere Heteroatome ausgewählt aus O, N und S beinhalten kann, und der mit einem Benzolring annelliert sein kann.

2. Verbindungen der Formel IV oder IVa, wie in Anspruch 1 definiert, worin Het für 2-Pyridyl oder 2-Benzthiazolyl steht.

3. Verfahren zur Herstellung von Verbindungen der Formel IV und IVa, wie in Anspruch 1 definiert, dadurch gekennzeichnet, dass man eine Verbindung der Formel
R₁ - COOH VI
worin R₁ die in Anspruch 1 angegebene Bedeutung besitzt, verestert.

4. Verfahren nach Anspruch 3, worin die Veresterung durch Reaktion mit 2,2'-Dithiopyridin oder Bis[benzthiazolyl-(2)]disulfid durchgeführt wird.

## Claims

1. Cephalosporin intermediate products of formula IV or IVa 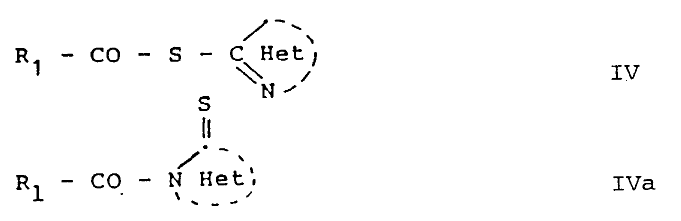 in which R₁ denotes a group of formula 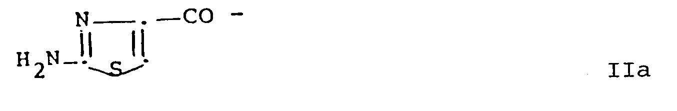 and 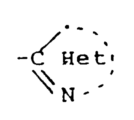 and 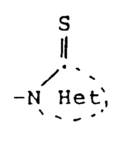 denote a 5- or 6-membered heterocycle, which in addition to the N-atom may contain one or two further hetero atoms selected from O, N and S, and which may be fuzed with a benzene ring.

2. Compounds of formula IV or IVa, as defined in claim 1 wherein Het denotes 2-pyridyl or 2-benzothiazolyl.

3. Process for the production of compounds of formula IV and IVa as defined in claim 1, characterized in that a compound of formula
R₁ - COOH VI
wherein R₁ possesses the definition given in claim 1, is esterified.

4. Process according to claim 3, wherein esterification is effected by means of a reaction with 2,2'-dithiopyridine or bis[benzothiazol-2-yl]disulphide.

## Revendications

1. Produits intermédiaires de la céphalosporine de formule IV ou IVa 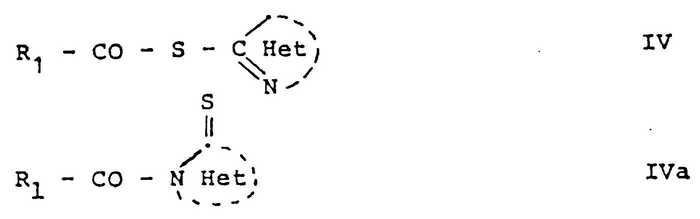 dans lesquelles R₁ signifie le groupe de formule 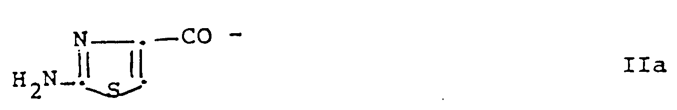 et respectivement 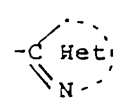 et 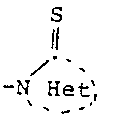 représentent un hétérocycle à 5 ou 6 chaînons qui peut contenir, en plus de l'atome d' azote, un ou deux autres hétéroatomes choisis parmi O, N et S, et qui peut être condensé avec un cycle benzénique.

2. Les composés de formule IV ou IVa selon la revendication 1, dans lesquels Het signifie un groupe 2-pyridyle ou 2-benzothiazolyle.

3. Procédé de préparation des composés de formule IV et IVa selon la revendication 1, caractérisé en ce qu'on estérifie un composé de formule
R₁ - COOH (VI)
où R₁ a la signification donnée à la revendication 1.

4. Un procédé selon la revendication 3, dans lequel l'estérification est effectuée par réaction avec la 2,2'-dithiopyridine ou le disulfure de bis[benzothiazole-2-yle].
